# DEMANDE DE BREVET EUROPEEN

(11) **EP 2 514 470 A1**
(43) Date de publication de la demande: **24.10.2012**
(21) Numéro de dépôt: 12290135.8
(22) Date de dépôt: 18.04.2012
(51) Int. Cl.: A61M 16/04, A61M 16/08, A61F 5/56, A62B 9/06, B63C 11/18

(54) **Dispositif pour lutter contre le stress**

(30) Priorité: 21.04.2011 FR 1101251
(71) Demandeur: Lopez, Laurent, 64600 Anglet (FR)
(72) Inventeur: Lopez, Laurent, 64600 Anglet (FR)

(57) **Abrégé**

Dispositif pour lutte contre le stress en s'aidant de la respiration.

L'invention concerne un dispositif permettant de mieux ressentir sa respiration pour développer des méthodes de lutte contre le stress sans entrainer de contraction musculaires sur les muscles du visage et du cou.

Il est constitué d'un embout (1) présentant un orifice (3) permettant l'écoulement de l'air en dehors de la bouche. D'une collerette (2) à l'une des extrémités de l'embout (1) permettant le maintient sans efforts du dispositif. Lorsque l'utilisateur respire avec l'embout la résistance apportée par l'orifice (3) lui permet de mieux ressentir sa respiration et de développer une sensation qui lui permettra de diminuer son stress.

Le dispositif selon l'invention est particulièrement destiné à lutter contre le stress.

## Description

La présente invention concerne un dispositif pour lutter contre le stress en s'aidant de la respiration.

La respiration thoracique ou abdominale est traditionnellement utilisée comme base dans les techniques de lutte contre le stress mais elle est, soit complètement libre et sans aide dans la bouche soit les aides apportées sont des embouts maintenus en bouche, par la pression des lèvres ou par un serrage entre les dents. Ces aides entrainent une tension musculaire sur les muscles du visage et le cou, entrainant ainsi une tension incompatible avec la lutte contre le stress.

Le dispositif selon l'invention permet de remédier à cet inconvénient. Il comporte en effet selon une première caractéristique, un embout présentant un orifice par lequel l'air peut s'écouler pendant le cycle respiratoire de l'utilisateur. La collerette permet de maintenir l'embout dans l'espace entre les dents et les lèvres sans effort. Cette collerette comporte des courbures permettant une meilleure étanchéité, en évitant les fuites d'air pendant la respiration. Elle permet également de maintenir centré l'orifice de l'embout. Par ses caractéristiques, cette collerette permet de maintenir l'embout dans la bouche de l'utilisateur pendant la respiration sans qu'aucunes contractions musculaires ne viennent gêner le travail de concentration et la mise en place de méthodes de lutte contre le stress. De plus cette collerette est adaptée à la courbure des dents pour améliorer le confort de l'utilisateur, lui permettant ainsi de ressentir sa respiration et de progresser dans les techniques de concentration et de méditation pour lutter contre le stress. La surface de l'orifice (3) du tube par lequel l'air s'écoule est de dimensions variables et elle fabrique une résistance à la respiration.

Selon les modes particuliers de réalisation :
- L'orifice par lequel souffle l'utilisateur et qui offre une résistance à l'expiration peut avoir différentes formes.
- L'embout qui aide à l'écoulement de l'air en dehors de la cavité buccale peut avoir une forme et une longueur différentes.
- La collerette peut comporter une surépaisseur (5) à sa périphérie, sur la face interne en contacte avec les dents afin de limiter le contacte dentaire.
- Le diamètre du tube peut être réduit en introduisant une pièce annexe (4) dans le conduit.
- La forme de la collerette peut être adaptée plus précisément par moulage sur la dentition du patient.

Les dessins annexés illustrent l'invention :
La figure 1 représente en coupe, le dispositif de l'invention.
La figure 2 représente en coupe, une variante de ce dispositif.

En référence à ces dessins, le dispositif comporte un embout (1) et un orifice (3) permet l'écoulement de l'air pendant le cycle respiratoire. A l'une de ses extrémités, l'embout (1) comporte une collerette (2) assurant le maintien et l'étanchéité pendant la respiration.

La forme de la collerette est telle que le dispositif tient sans efforts. La surface de l'orifice (3) et la longueur de l'embout sont telles, qu'ils génèrent une faible résistance pendant le cycle respiratoire de l'utilisateur.

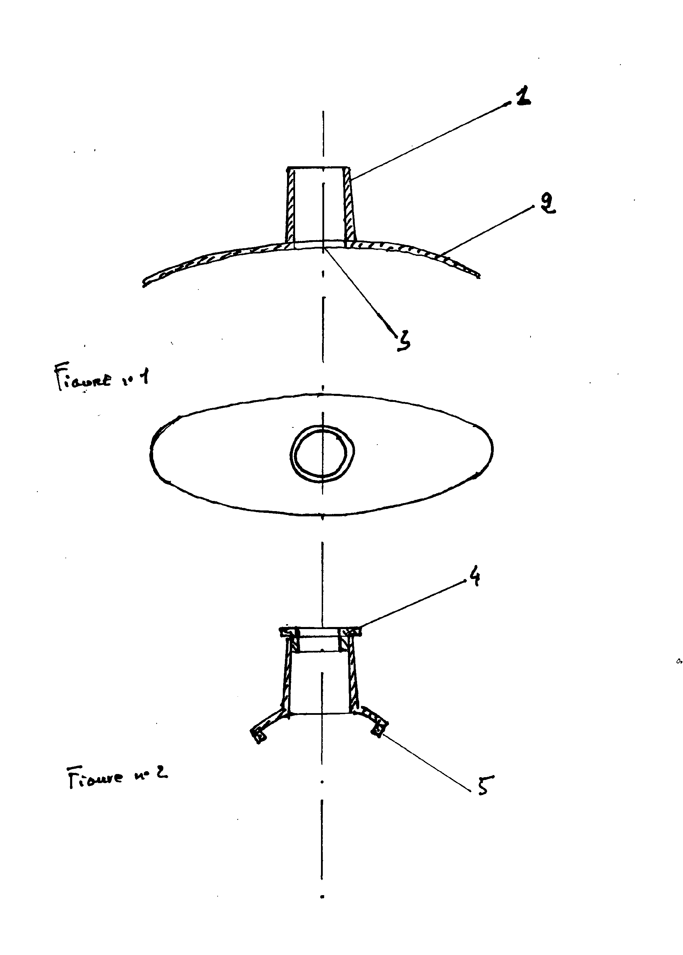

Dans la forme de réalisation selon la figure 2, la collerette (2) est de forme ellipsoïde pour limiter les fuites d'air pendant le cycle respiratoire. Cette collerette comporte des courbes pour s'adapter à la courbure des dents de telle sorte que le dispositif est maintenu entre les lèvres et les dents sans efforts musculaires. La collerette (2) présente une surépaisseur à sa périphérie sur sa face interne pour diminuer la surface de contacte entre les dents et le dispositif.

L'embout (1) est de longueur suffisante pour que l'air puisse s'écouler en dehors de la cavité buccale. L'axe de l'embout est incliné vers le bas.

L'orifice (3) est de forme cylindrique et une pièce annexe peut venir diminuer son diamètre.

A titre d'exemple non limitatif, le dispositif aura des dimensions de l'ordre de : Pour la collerette, 2 cm de largeur et 5 cm de long. Pour l'embout, le diamètre de l'embout sera compris entre 5 et 12 mm.

Le dispositif de la présente invention peut être réalisé en carton ou en matière synthétique (par exemple en silicone de qualité médicale) en une seule opération de moulage par injection.

Le dispositif selon l'invention est particulièrement destiné aux méthodes de lutte contre le stress basées en partie sur la respiration.

## Revendications

1. Dispositif pour lutter contre le stress **caractérisé en ce qu'**il comporte un embout (1) présentant un orifice (3) dans lequel l'air s'écoule et une collerette (2) qui permet l'étanchéité aux fuites d'air pendant les temps respiratoires et le maintien du dispositif dans la bouche, entre les lèvres et les dents, sans contraction musculaire des muscles de la face et du cou.

2. Dispositif selon la revendication 1 **caractérisé en ce que** la collerette (2) présente des courbures pour s'adapter à la forme de dents.

3. Dispositif selon l'une quelconque des revendications précédentes **caractérisé en ce que** l'embout canalise l'écoulement de l'air et constitue une résistance pendant les temps d'inspiration et d'expiration, l'aire de l'orifice (3) de l'embout (1) est à titre indicatif comprise entre 5 et 12 mm.
